Europäisches Patentamt

European Patent Office

Office européen des brevets

(11) Veröffentlichungsnummer: **0 363 622**
**A1**

# (12) EUROPÄISCHE PATENTANMELDUNG

(21) Anmeldenummer: 89116075.6

(22) Anmeldetag: 31.08.89

(51) Int. Cl.5: **C12N 9/64 , C12N 15/58 , A61K 37/547**

Patentansprüche für folgenden Vertragsstaat: ES.

Die Bezeichnung der Erfindung wurde geändert (Richtlinien für die Prüfung im EPA, A-III, 7.3).

(30) Priorität: 09.09.88 DE 3830734

(43) Veröffentlichungstag der Anmeldung:
18.04.90 Patentblatt 90/16

(84) Benannte Vertragsstaaten:
AT BE CH DE ES FR GB IT LI NL SE

(71) Anmelder: BASF Aktiengesellschaft
Carl-Bosch-Strasse 38
D-6700 Ludwigshafen(DE)

(72) Erfinder: Bollschweiler, Claus, Dr.
Karl-Christ-Strasse 13
D-6900 Heidelberg(DE)
Erfinder: Schmidt, Martin, Dr.
Wichernstrasse 24
D-6803 Edingen-Neckarhausen(DE)
Erfinder: Strube, Karl-Hermann, Dr.
Kurt-Schumacher-Strasse 49 a
D-6720 Speyer(DE)
Erfinder: Baldinger, Verena, Dr.
Schiffsgasse 6
D-6900 Heidelberg(DE)

(54) **Polypeptide mit Plasminogenaktivität, ihre Herstellung und Verwendung.**

(57) Es werden Polypeptide der Formel
$tPA_{1-54}$-X-$tPA_{172-527}$ ,
worin $tPA_{1-54}$ und $tPA_{172-527}$ die in der Beschreibung angegebene Bedeutung besitzen, und deren Herstellung beschrieben. Die neuen Polypeptide eignen sich zu Bekämpfung von Krankheiten.

EP 0 363 622 A1

## Neue Polypeptide, ihre Herstellung und Verwendung

Die vorliegende Erfindung betrifft neue Polypeptide mit Plasminogenaktivatoraktivität, Verfahren zu deren Herstellung und deren Verwendung bei der Bekämpfung von Krankheiten.

Der humane, gereifte Gewebe-Plasminogenaktivator (tPA) ist ein Polypeptid aus 527 Aminosäuren und einem Molekulargewicht von etwa 68 kD (Pennica et al. 1983, Nature 301, 214 - 221 und Ny et al. 1984, Proc. Natl. Acad. Sci. USA 81, 5355-5359) (vgl. Abb. 1a-1c). Das Molekül enthält mehrere distinkte Regionen, sog. Domänen, denen verschiedene Funktionen zugeordnet werden. Der N-Terminus wird durch eine Finger-Region gebildet, die dem Fibronektin homolog ist. Anschließend folgt eine Region, die Ähnlichkeit mit mehreren Wachstumsfaktoren besitzt. Im Anschluß daran folgen zwei Kringel-Domänen. Nach einer Spaltstelle, an der das einkettige Molekül in zwei Ketten gespalten werden kann, schließt sich die Protease-Domäne an; diese enthält das aktive Zentrum und besitzt Homologie zu anderen Serin-Proteasen.

Mehrere Eigenschaften machen den Gewebe-Plasminogenaktivator zu einem interessanten Molekül: tPA besitzt eine starke Affinität zu Fibrin, die Plasminogen-Aktivierung ist Fibrin-abhängig; als Protease spaltet tPA Plasminogen zu Plasmin, das wiederum Fibrin zu Spaltprodukten abbaut. tPA ist durch Plasminogenaktivator-Inhibitoren hemmbar. Ferner hat tPA eine relativ kurze Halbwertzeit; das Molekül wird in der Leber rasch abgebaut.

Diese Faktoren bewirken, daß tPA in vivo spezifisch an Blutgerinnseln Plasminogen zu Plasmin aktiviert und durch Abbau des Fibrins die Wiederherstellung des Gefäßstromes bewirkt. tPA kann deshalb in der fibrinolytischen Therapie, z. B. nach einem Herzinfarkt, eingesetzt werden.

Es wurde nun gefunden, daß Polypeptide der Formel

$tPA_{1-54}$-X-$tPA_{172-527}$ ,

worin

$tPA_{1-54}$ und $tPA_{172-527}$ die Aminosäuren 1-54 und 172-527 des humanen, gereiften Gewebe-Plasminogenaktivators und

X eine der Aminosäuresequenzen

- Ser-$tPA_{111-171}$,
- $tPA_{55-105}$,
- $tPA_{55-130}$-Arg-$tPA_{140-171}$ oder
- $tPA_{55-125}$-$tPA_{139-171}$

bedeuten, wobei $tPA_{111-171}$, $tPA_{55-105}$, $tPA_{55-130}$, $tPA_{140-171}$, $tPA_{55-125}$ und $tPA_{139-171}$ die Aminosäuren 111-171, 55-105, 55-130, 140-171, 55-125 und 139-171 des humanen, gereiften Gewebeplasminogenaktivators sind, sowie deren Allelvarianten oder Derivaten mit am N- und/oder C-Terminus um ein bis sechs Aminosäuren verkürzten oder verlängerten Sequenzen verbesserte Eigenschaften besitzen.

Die neuen Polypeptide sind also zwischen den Aminosäuren 54 und 172 des tPA verändert.

Die Erfindung betrifft weiter DNA-Sequenzen, die für die mutierten Polypeptide codieren, sowie Vektoren, die diese DNA-Sequenzen enthalten.

Die neuen Polypeptide lassen sich gentechnisch nach bekannten Verfahren herstellen. Ausgangspunkt für die hier beschriebenen Konstruktionen ist ein cDNA-Klon, im folgenden pCUtpa genannt, der die codierende Sequenz von tPA und noch 5'- und 3'- nicht translatierte Bereiche enthält. Nach einer "leader"- und Prosequenz beginnt das reife Protein mit Ser(1) und endet nach Pro(527).

pUCtPA wird die folgt isoliert: Aus menschlichem Uterus-Gewebe wird mRNA isoliert und in doppelsträngige cDNA umgeschrieben wird. Nach Einsetzen dieser cDNA in den kommerziell erhältlichen Klonierungsvektor pUC9 wird eine cDNA-Bibliothek angelegt. Die dabei verwendeten Methoden sind beispielsweise in Maniatis et al., Molecular Cloning, CSH-press, nachzulesen. Auch das "screening" solcher Genbanken mit radioaktiv markierten Oligonukleotidsonden ist inzwischen ein vielfach verwendete und beschriebene Methode. Nach diesem Verfahren kann ein cDNA-Klon, der die kodierende Region und angrenzende Bereiche enthält, isoliert werden.

Teile der DNA-Sequenz von PUC9tPA sind mit Hilfe von Restriktionsenzymen leicht zugänglich. Diese Fragmente, ggf. in Verbindung mit chemisch synthetisierten Oligonukleotiden, Adaptoren oder Genfragmenten können benutzt werden, um die DNA-Sequenzen, die für die neuen Polypeptide codieren, zu klonieren. Der Einbau der Genfragmente bzw. synthetischen DNA-Sequenzen in Klonierungsvektoren, z.B. die handelsüblichen Plasmide pBR322, pUC8 oder 9, pCU18 oder 19, M13mp18 oder M13mp19 erfolgt in bekannter Weise. Auch können die Gene oder Genfragmente mit geeigneten chemisch synthetisierten oder aus Bakterien, Phagen, Eukaryontenzellen oder deren Viren isolierten Kontrollregionen versehen werden, die die Expression der Proteine ermöglichen. Die Transformation bzw. Transfektion der so erhaltenen Hybridplasmide in geeignete Wirtsorganismen ist ebenfalls bekannt und eingehend beschrieben. Auch können die Hybridplasmide mit entsprechenden Signalsequenzen versehen werden, die die Sekretion der Polypeptide ins Medium erlauben.

Bei der Expression in Säugerzellen kann man Vektoren verwenden, die das zu exprimierende Gen, in diesem Fall die mutierte tPA-cDNA, unter die Kontrolle des Maus-Methallothionein- oder des viralen SV40-Promoters setzt. Notwendig für die Expression ist das Vorliegen des Methionin-Startcodons und der Leader-/Prosequenz des tPA-Gens. Man isoliert dann Klone, die Kopien dieser Vektoren als Episome oder ins Genom integriert besitzen. Besonders vorteilhaft sind z. B. die Integration und Expression des Fremdgens auf der Basis des Rinderpapillom-Virus. In Verbindung mit prokaryontischen Sequenzen, die für die Replikation in Bakterienzellen und eine Antibiotika-Resistenz codieren, ist der Aufbau sog. "shuttle"-Vektoren möglich. Konstruktion und Vermehrung des Plasmids erfolgen zunächst in Bakterienzellen; anschließend erfolgt die Umsetzung in die Eukaryontenzellen, z.B. in die Maus-Fibroblastenzellinie C127. Es ist selbstverständlich, daß auch andere Zellsysteme, z.B. Hefe, Insektenzellen und andere Säugerzellen, wie z.B. CHO-, L- und 293-Zellen, für die Expression verwendet werden können.

Diese eukaryontischen Expressionssysteme besitzen den Vorteil, daß sie in der Lage sind, ihre Produkte effektiv und meist in nativer Form zu sezernieren. Ferner besitzen sie die Fähigkeit, ihre Produkte posttranslational zu modifizieren.

So erhält der Gewebe-Plasminogenaktivator bei der Expression in Eukaryontenzellen noch Glykosidseitenketten an den Aminosäuren 117, 184 und 448 (Asn). Bakterien sind nicht in der Lage, Glycosidseitenketten zu synthetisieren. Die meisten in Bakterien exprimierten eukaryonitischen Proteine, wie auch tPA und die von ihr erfindungsgemäß abgeleiteten Polypeptide, fallen in der Zelle als denaturierte Einschlußkörper an und müssen proteinchemisch renaturiert werden. Ferner sind Bakterien oft nicht in der Lage, die Initiatoraminosäure Methionin vom fertigen Protein abzuspalten. Durch die Verwendung von Sekretionssystemen können diese Schwierigkeiten umgangen werden.

Aufgrund der Degeneration des genetischen Codes ist es aber auch möglich, andere DNA-Sequenzen, z.B. chemisch synthetisierte Gene mit unterschiedlicher DNA-Sequenz für die Expression der neuen Polypeptide zu benutzen.

Die Reinigung der neuen Polypeptide erfolgt durch deren Abtrennung aus dem Kulturmedium durch Affinitäts- und Ionenaustausch-Chromatographie nach bekannten Verfahren.

Die erfindungsgemäß erhaltenen Polypeptide besitzen eine erhöhte Gerinnselspezifität, längere Halbwertzeit, verringerte Inhibitorbindung und/oder größere proteolytisch Aktivität und können daher zur Thrombolyse eingesetzt werden. Sie zeigen dabei verbesserte Eigenschaften gegenüber humanem Gewebe-Plasminogenaktivator.

Gegenstand der Erfindung sind daher auch Arzneimittel, die mindestens eines der neuen Polypeptide enthalten, ggf. in einem pharmazeutisch verträglichen Träger oder Bindmittel. Die Arzneimittel können auch Kombinationen der neuen Proteine mit anderen Fibrinolytika, wie Prourokinase, Urokinase oder Streptokinase oder deren Derivate, sowie mit anderen Pharmaproteinen, z. B. Superoxiddismutase enthalten. Weitere Ausgestaltungen der Erfindung sind in den folgenden Beispielen näher beschrieben.

Für gentechnische Methoden sei dazu z. B. auf das Handbuch von Maniatis et al., Molecular Cloning, Cold Spring Harbor Laboratory, 1982, verwiesen.

Beispiel 1

Isolierung eines cDNA-Klones für humanen Gewebe-Plasminogenaktivator

30 g tPA-produzierendes menschliches Uterus-Gewebe wurde in 6 M Guanidiniumthiocyanat, 5 mM Natriumcitrat (pH 7,0), 0,1 M 2-Mercaptoethanol, 0,5 % Sarcosyl im Ultra-Turax® aufgeschlossen. Grobe Zelltrümmer wurden bei 3000 rpm abzentrifugiert. Die RNA wurde durch Zentrifugation durch eine 5,7 M CsCl-Kissen über Nacht bei 45.000 rpm abgetrennt. Anschließend wurde die polyA$^+$-enthaltende RNA-Fraktion durch Affinitätschromatographie an oligo(dT)-Cellulose abgetrennt.

Mit Hilfe des Enzyms AMV-Reverse Transcriptase und oligo(dT)12-18 als Starter wurde die polyA$^+$-RNA in einsträngige cDNA umgeschrieben. Die Synthese des zweiten Stranges erfolgte mit E. coli-DNA-Polymerase I. An die doppelsträngige cDNA wurden mit Hilfe des Enzyms T4-DNA-Ligase SalI-Linker angesetzt. Das kommerziell erhältliche Plasmid pUC9 wurde mit dem Restriktionsenzym SalI linearisiert. Beide DNAs wurden miteinander ligiert und mit dem so erhaltenen Hybrid C aCl$_2$-behandelte, kompetente Zellen des E. coli-Stammes HB101 transformiert.

Die Zellen wurden auf LB-Platten mit 100 μg/ml Ampicillin plattiert und über Nacht bei 37°C inkubiert.

Die Kolonien wurden auf Nitrocellulose-Filter übertragen, Replikaplattiert, mit 0,5 N NaOH/1,5 M NaCl lysiert und die denaturierte DNA durch 2-stündiges Backen bei 80°C fest an den Filter gebunden.

Die Filter wurden in 6 x SET-Puffer (1 x SET = 0,15 M NaCl, 15mM Tris/HCL pH 7,4, 1 mM EDTA), 0,1 % SDS und 5 x Denhardt's Lösung (100 x Denhardt = 1 g Ficoll, 1 g Polyvinylpyrrolidon, 1 g BSA pro 50 ml) für 4 h, bei 68°C vorhybridisiert. Mit Hilfe eines DNA-Synthesizers wurden drei Oligonukleotid-Sonden, die jeweils 17 Basen der tPA-DNA umfassen, hergestellt. Sie bestehen aus folgenden Sequenzen:
5′ TGCAGATCACTTGGTAA 3′

5' CCAGGCCCAGTGCCTGG 3'
5' TCCAGTCCGGCAGCTGC 3'

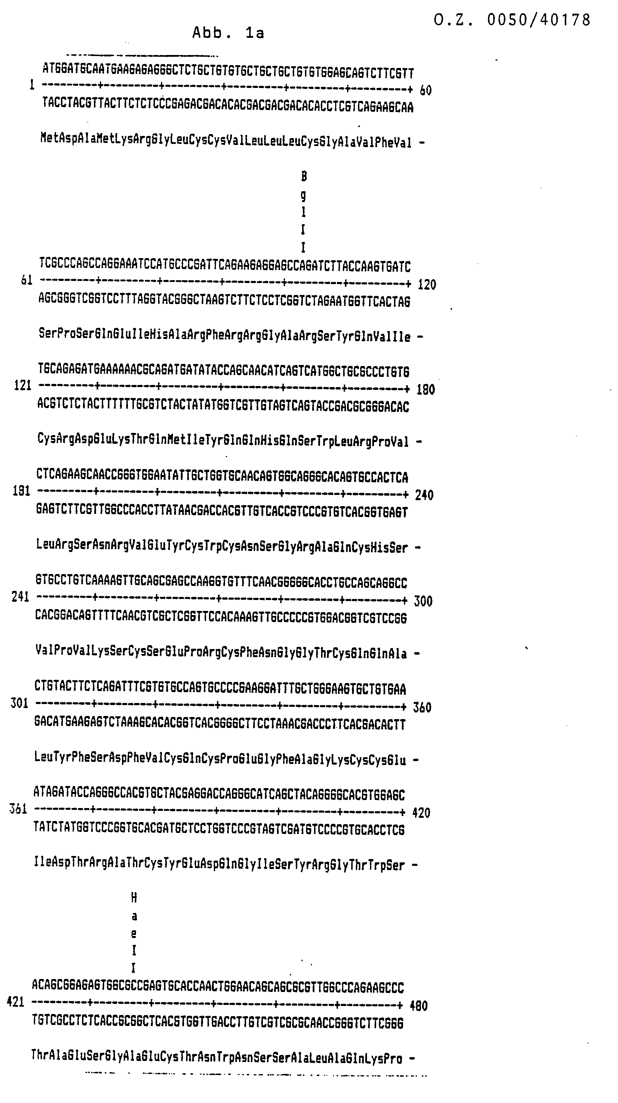

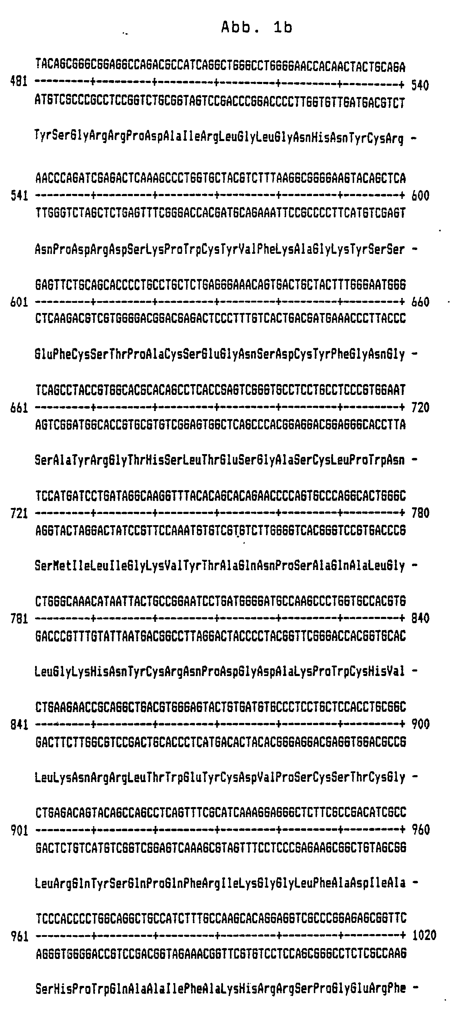

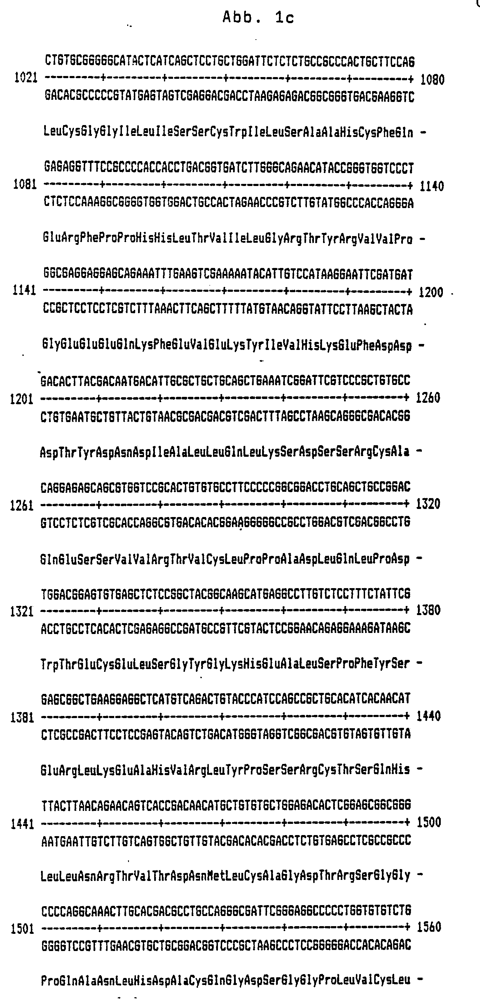

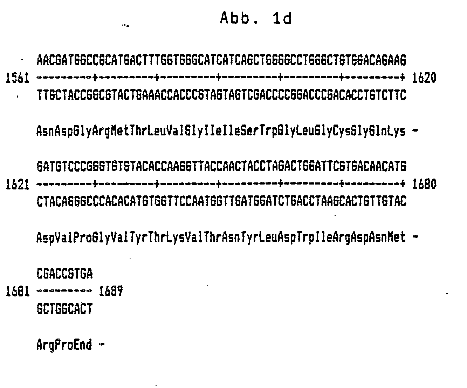

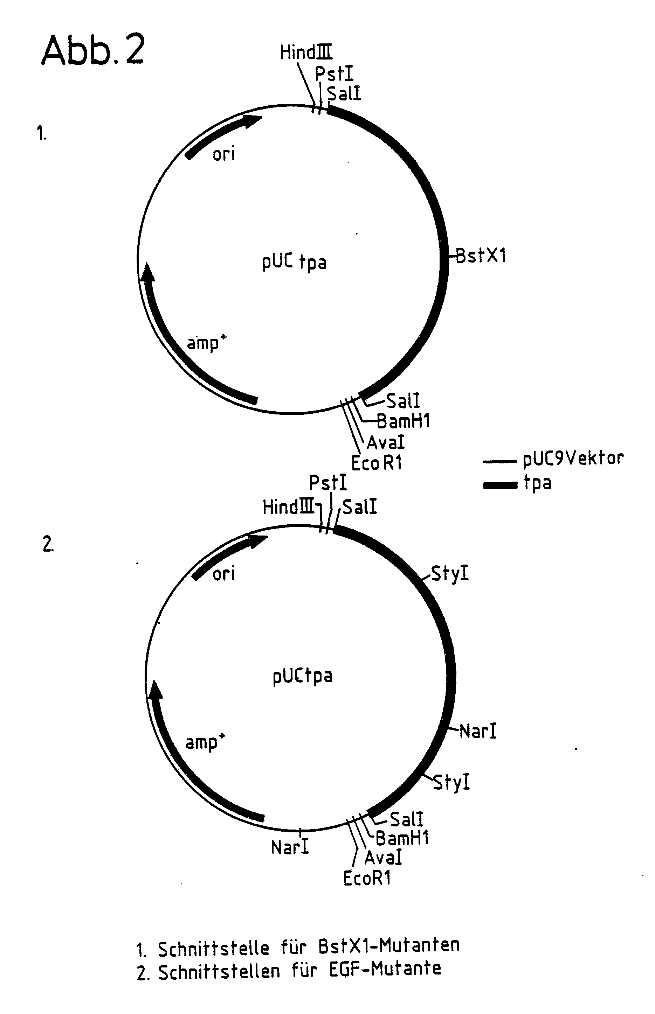

Diese Sonden wurden am 5'-Ende mit γ-³²P-ATP markiert. Sie wurden dann mit den vorhybridisierten Filtern in einer Lösung, die 6 x SET, 0,1 % SDS, 5 x Denhardt's und 10 % Dextransulfat enthält, über Nacht bei 42° C unter leichtem Schütteln inkubiert. Die Filter werden danach mehrfach in 6 x SET/0,1 % SDS bei 42° C gewaschen, angetrocknet und einem Röntgenfilm exponiert. Klone, die beim "Screening" eine radioaktive Antwort gaben, wurden isoliert und weitergezüchtet. Ein Klon, im folgenden pUCtPA genannt, enthält ein etwa 2,1 kb großes Insert, das die codierende Region, sowie 5'- und 3'-nicht-codierende Bereiche enthält (Abb. 2). Plasmid-DNA von pUCtPA wurde durch Lysozym-Aufschluß und SDS-Alkali-Behandlung der Bakterienkultur sowie anschließende CsCl-Gradientenzentrifugation präpariert.

Beispiel 2

Deletion von Nukleotiden aus der tPA-DNA

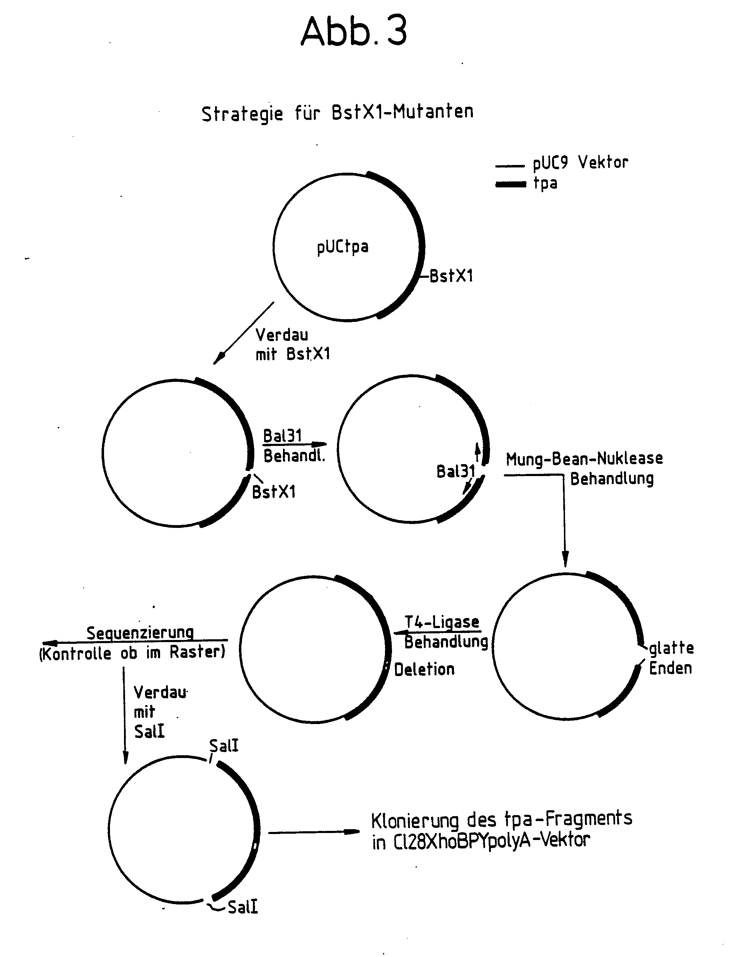

Ausgangsprodukt war das Plasmid pUC tPA (Abb. 2). Dieses wurde präparativ mit der Restriktionsendonuklease BstX1 geschnitten. Mit der Exonuklease Bal31 wurde die DNA in verschiedenen Ansätzen zwischen 0 und 10 min behandelt (Abb. 3). Die Reaktionen wurden jeweils mit EGTA abgestoppt. Die DNAs wurden mit Alkohol gefällt und gewaschen und danach mit Mung Bean Nuklease behandelt (glatte Enden). Nach einer Phenol/Chloroform-Extraktion und nochmaliger Alkoholfällung wurden die DNAs mit T4-Ligase rückligiert und in E.coli CMK603 transformiert.

Aus den Transformanten wurden Plasmide isoliert und mit EcoR1 geschnitten. Es konnten so tPA-Gene unterschiedlicher Größe identifiziert werden. Mehrere tPA-Gene mit unterschiedlich großen Deletionen wurden sequenziert, um das Leseraster (Aminosäuresequenz) zu bestimmen. Deletionsmutanten mit durchgehendem Leseraster wurden in das Plasmid pCL28Xho BPV (siehe Beispiel 4) umkloniert, in Mausfibroblasten exprimiert und getestet.

Beispiel 3

Deletion von Nukleotiden aus der tPA-DNA

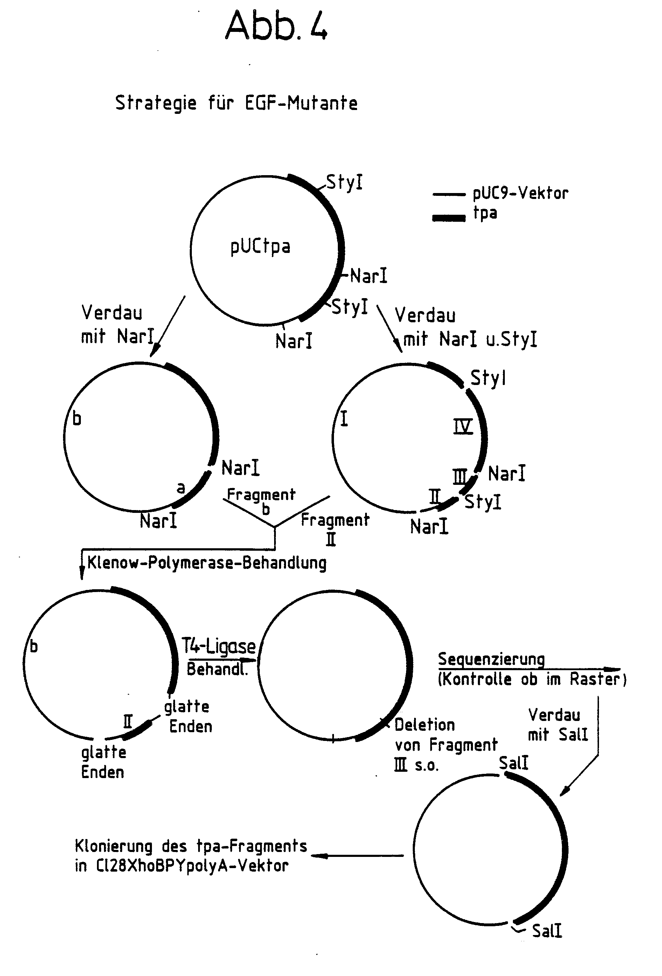

Ausgangsprodukt war das Plasmid pUC tPA (Abb. 2). Dieses wurde in einem Ansatz präparativ mit NarI geschnitten. In einem zweiten Ansatz wurde des präparativ mit NarI und StyI doppelt geschnitten (Abb. 4). Vom ersten Ansatz wurde die größere Bande (ungefähr 4150 Basenpaare = Vektorbande +3'-Ende tPA) aus einem Agarosegel isoliert.

Vom zweiten Ansatz wurde die 457-Basenpaare große Bande aus einem Agarosegel isoliert (= NarI/SalI-Fragment vom Vektor und 5'-Ende vom tPA).

Die beiden Fragmente wurden anschließend mit Klenow-Polymerase behandelt und die 5'-überhängenden Enden zu glatten Enden aufgefüllt. Dadurch wurde sichergestellt, daß das verkürzte tPA-Gen im Leseraster blieb. Nach einer Phenol/Chloroform-Extraktion und anschließender Alkoholfällung wurden die beiden Fragmente mit T4-Ligase zusammenligiert und in E.coli CMK 603 transformiert. Von einigen Transformanten wurden Plasmide präpariert und mit Restriktionsenzymen (z.B. Ava1) analysiert. Die DNA eines verkürzten tPA-Gens wurde zur Kontrolle sequenziert und dann in das Plasmid pCL28Xho BPV (siehe Beispiel 4) umkloniert, in Mausfibroblasten exprimiert und getestet.

Beispiel 4

Konstruktion von Vektoren für die Expression der modifizierten tPA-DNA

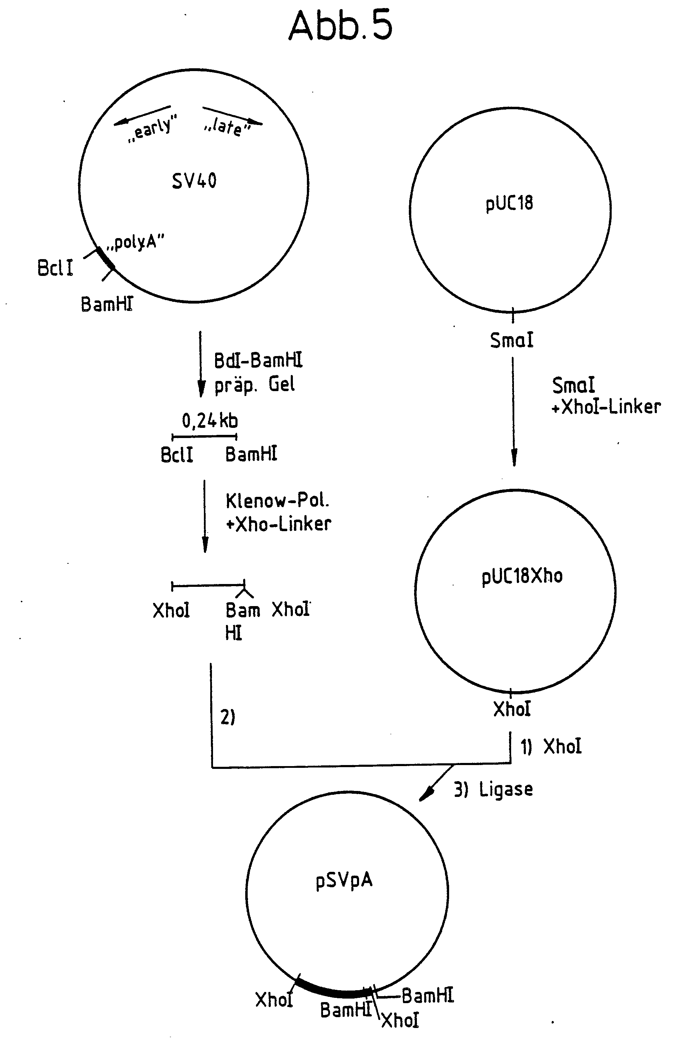

DNA des Affenvirus SV40 wurde mit den Restriktionsenzymen BamHI und BclI geschnitten und das 0,24 kb-Fragment gelelektrophoretisch präpariert (Abb. 5). Die Enden wurden in Gegenwart der vier Desoxynukleotidtriphosphate dATP, dCTP, dGTP und dTTP mit dem Klenow-Fragment der DNA-Polymerase I aufgefüllt. Anschließend wurden XhoI-Linker anligiert.

Parallel wurde der kommerziell erhältliche Vektor pUC18 mit dem Enzym SmaI linearisiert. Dann wurden ebenfalls XhoI-Linker angesetzt. DNA dieses Vektors ("pUC18Xho") wurde mit XhoI linearisiert, mit alkalischer Phosphatase behandelt und mit dem 0,24 kb XhoI-SV40-Fragment (s. o.) ligiert. Es entstand pSVpA.

pSVpA-DNA wurde präparativ mit XhoI gespalten und wie oben mit Klenow-Polymerase in Gegenwart der vier dNTPs inkubiert. Das 0,24 kb-Fragment wurde Gel-isoliert.

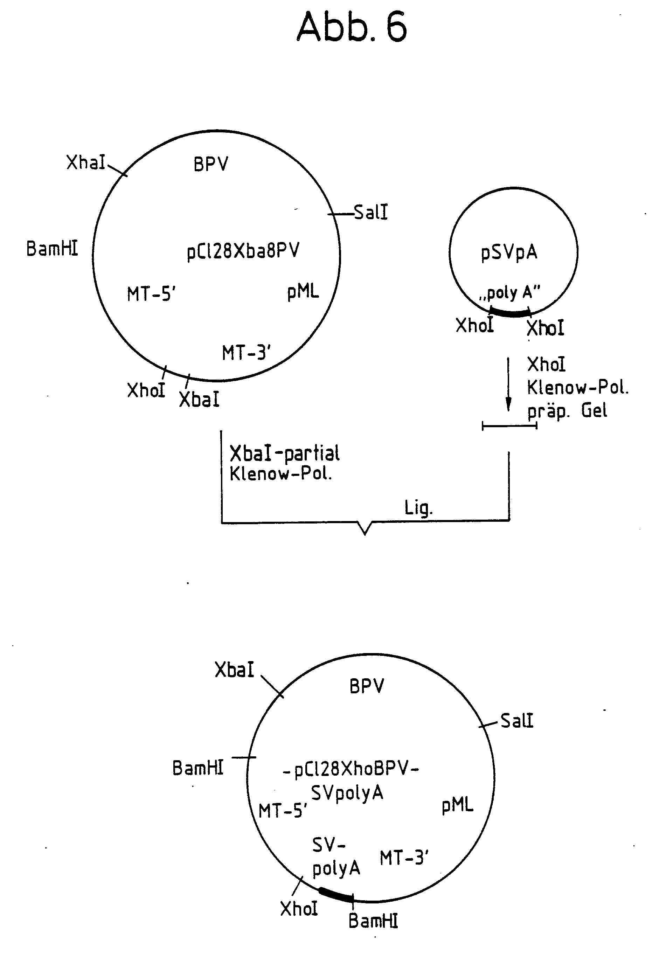

Gleichzeitig wurde der Eukaryonten-Expressionsvektor CL28XhoBPV, entstanden durch Ligation von CL28X und pB2-2 (nach Reddy et al. 1987, DNA 6, 461-72), partiell mit dem Restriktionsenzym XbaI geschnitten, d. h. es wurde zeitlich derart limitiert inkubiert, daß Moleküle entstanden, die nur an einer der beiden XbaI-Erkennungssequenzen gespalten, also linearisiert sind (Abb. 6). Der Ansatz wurde dann wie beschrieben mit Klenow-Polymerase und dNTPs umgesetzt. Die linearen Moleküle wurden anschließend durch Gelelektrophorese isoliert. -

Es erfolgte dann die Ligation der linearen pCL28XhoBPV-Fragmente mit dem vorbehandelten 0,24 kb-Fragment aus SV40. Nach Transformation und Screening von Minilysaten wurde ein Klon isoliert, der das SV40-Fragment in der etwa 0,15 kB 3′-wärts der XhoI-Stelle gelegenen vormaligen XbaI-Stelle trug; diese DNA ("pCL28XhoBPV-SVpolyA") trug die SV40-Transkriptionsstopsignale der "frühen" Gene.

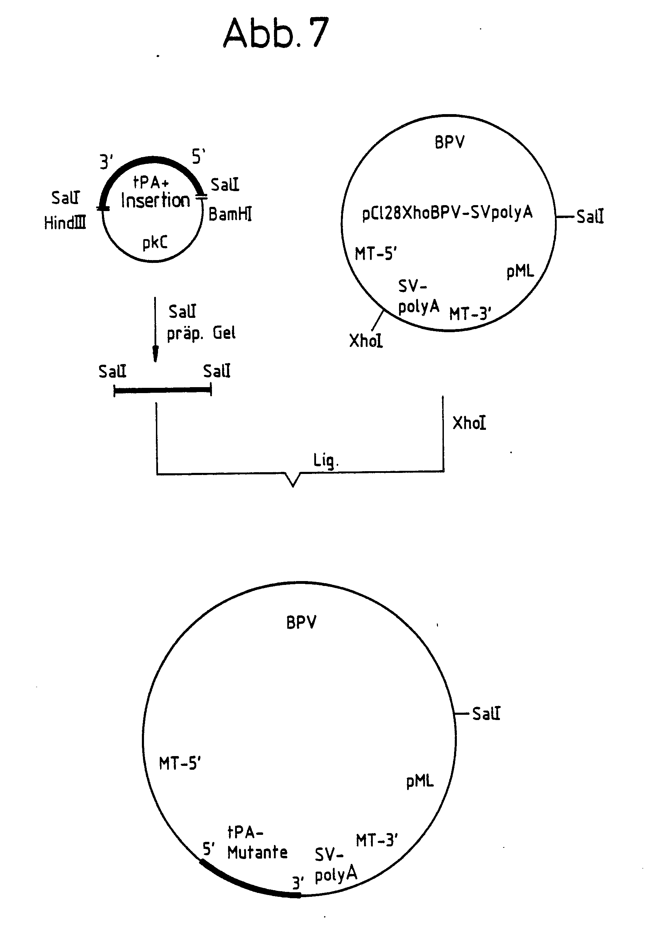

Plasmid-DNA von pCL28XhoBPV-SVpolyA wurde mit dem Restriktionsenzym XhoI linearisiert und mit alkalischer Phosphatase behandelt. Gleichzeitig wurde pUCtPA-CTX, -CTN, -CTT, -CTFg, CTaF, -CTV (aus den Beispielen 2 und 3) mit dem Enzym SalI gespalten und das ca. 2,1 kb große Insert präpariert (Abb. 7). Beide Fragmente wurden mittels T4-Ligase miteinander verbunden. Nach Transformation und Minilysaten wurde ein Klon isoliert, der die mutierte tPA-DNA einfach und in der korrekten Orientierung enthielt: pCL28BPV-tPA-CTX, -CTN, -CTT, -CTFg, -CTaF, -CTV.

Beispiel 5

Transfektion und Etablierung von Zellinien

C127I Zellen (J. Virol. 26 (1978) 292; ATCC catalogue of cell lines and hybridomas 5th edition, 1985, p142) wurden mit BPV-Expressionsplasmiden transfiziert mit der Calciumphosphat-Copräzipitationsmethode (Virology 52 (1973)/ 456, DNA cloning; volume II; ed. D.M.Glover IRL Press, Seiten 143ff und 213 (1985)).
$5 \times 10^5$ C127I-Zellen wurden in DMEM (Dulbecco's Modified Eagles Medium) + 10 % FCS (Foetales Kalbsserum) in 60 mm Petrischalen eingesät. Am nächsten Tag wurde das Medium gewechselt auf MEM (Modified Eagles Medium) mit 25mM Hepes + 10 % FCS. Mit 10 µg CsCl-gereinigter Plasmid DNA wurde ein Ca-Phosphat-Corpräzipitat gebildet, welches vorsichtig auf die C127I-Zellen aufgebracht wurde. Die Zellen wurden 4 h bei 37° C; 7 % $CO_2$ inkubiert. Durch eine anschließende Glycerin-Schock-Behandlung wurde die Effizienz der Transfektion erheblich gesteigert. Hierzu wurde 4 h nach Aufbringen des Präzipitates das Medium von den Zellen abgezogen. Die Zellen wurden 3 min. mit je 2 ml 15 % Glycerin/HBS (DNA cloning Vol. II, Seite 152) pro 60 mm Petrischale bei Raumtemperatur inkubiert. Die Glycerin/HBS-Lösung wurde abgezogen, der Zellrasen mit 3 ml DMEM + 10 % FCS gewaschen. Die Zellen wurden mit DMEM + 10 % FCS bei 37° C; 7 % $CO_2$ inkubiert. Dreimal in der Woche wurde das DMEM + 10 % FCS abgezogen und durch frisches ersetzt. Nach 2-3 Wochen waren transfizierte Zellen, die das BPV-Genom enthielten, als Ansammlungen transformierte Zellen, sogenannte Foci, zu erkennen.

Foci, die die oben beschriebenen tPA-Muteine exprimieren, wurden durch einen Casein-Agar-Overlay (siehe unten) identifiziert. Nach 2-3 h Inkubation bei 37° C; 7 % $CO_2$ waren im trüben Casein-Agar Lysehöfe an den Stellen zu erkennen, an denen sich tPA-exprimierende Zellen befinden. Nach Entfernen des Casein-Agar wurden die sich an diesen Stellen befindenden Zellen nach der "cloning-cylinder"-Methode isoliert (DNA cloning Vol. II, S. 220). Die erhaltenen Zellinien wurden anschließend mit Standardmethoden in DMEM + 10 % FCS hochgezüchtet.

Zur Produktion wurden die Zellinien nach Erreichen der Konfluenz in serumfreiem DMEM gehalten. Das tPA-Mutein aus dem so erhaltenen serumfreien Zellkulturüberstand kann nun charakterisiert und aufgereinigt werden.

Für den oben erwähnten Agar Overlay Test werden folgende Lösungen benötigt:

Overlay-Agarose

1) 8 % Magermilchlösung in $H_2O$ werden 30 min bei 100° C gekocht und anschließend im 37° C Wasserbad abgekühlt.

2) Eine 2 %ige Lösung von Low-melting Agarose in PBS wird nach Autoklavieren im 37° C Wasserbad abgekühlt. Anschließend wird im Verhältnis 1:1 doppelt konzentriertes DMEM zugegeben.

3) 0,64 mg Plasminogen wird in 1 ml $H_2O$ gelöst. Durch Zusammenpipettieren von 16 ml Lösung 2, 4 ml Lösung 1 und 0,4 ml Plasminogen wird die Overlay Agarose hergestellt. Diese wird nach Mischen im 37° C Wasserbad gehalten.

Zur Durchführung des Tests werden die Zellen in 60 mm Petrischalen zweimal mit serumfreiem Medium gewaschen. Anschließend werden je 2 ml "Overlay-Agarose" vorsichtig in die Petrischalen pipettiert. Die Petrischalen werden zum Abkühlen und Erstarren der Agarose bei Raumtemperatur stehen gelassen. Anschließend wird 2-3 h im Brutschrank bei 37° C unter Zugabe von 7 % $CO_2$ inkubiert und die Größe und Anzahl der Lysehöfe bestimmt.

Beispiel 6

Isolierung eines neuen Polypeptids

Aus dem gemäß Beispiel 5 erhaltenen serumfreien Zellkulturüberstand wurde das tPA-Mutein nach Sterilfiltration und Zugabe von 0,01 % ®Tween 80 durch eine Affinitätschromatographie an Erythrina-Trypsin-Inhibitor-Sepharose (ETI-Sepharose, 1 cm x 3 cm) isoliert. Zur Herstellung der Affinitätsmatrix wurden 5 mg ETI an 1 ml CNBr-aktivierte Sepharose 4B gekoppelt. Das Gelmaterial wurde vor dem Auftragen des Zellüberstands mit 20 mM Na-phosphat, 0,15 M NaCl, 0,01 % ®Tween 80, pH 7,0 äquilibriert. Nach dem Auftragen wurde das Gelmaterial mit demselben Puffer behandelt, um unspezifisch gebundenes Material zu entfernen. Die Desorption des spezifisch gebundenen Muteins erfolgte anschließend durch Elution mit 0,1 M Glycin, 0,1 M Arginin/HCl, 0,01 % ®Tween 80, pH 3,0. Das Eluat wurde vereinigt und mit 0,1 N Natronlauge auf pH 5,0 eingestellt. Alternativ wurde das Eluat mit dem 3fachen Volumen an 20 mM Na-PO$_4$, 250 mM NaCl 0,01 % ®Tween 80 verdünnt und ein pH-Wert von 7 eingestellt. Zur weiteren Reinigung des isolierten Muteins wurde die Proteinlösung dann auf eine mit 20 mM Na-PO$_4$, 250 mM NaCl, 0,01 % ®Tween 80, pH 7,0 äquilibrierte Lysin-®Sepharose-Säule (1 cm x 7 cm) aufgetragen. Um unspezifisch gebundenes Material zu entfernen wurde mit 20 mM Na-PO$_4$, 0,01 % ®Tween 80, pH 6,5 gespült. Die Desorption des spezifisch gebundenen Muteins erfolgte dann mit 20 mM Na-PO$_4$, 0,4 M Arginin, 0,01 % ®Tween 80, pH 6,5. Abschließend wurde die Proteinlösung noch gegen 20 mM Na-PO$_4$; 0,1 M Arginin, 0,01 % ®Tween 80, pH 5,0 dialysiert und schließlich bei -20° C bis zur weiteren Verwendung aufbewahrt.

Beispiel 7

Charakterisierung des Oligosaccharid-Anteils

5 - 10 µg des gemäß Beispiel 6 erhaltenen Muteins wurden durch SDS-Gelelektrophorese aufgetrennt und anschließend auf Nitrozellulose-Membranen transferiert. Nach dem Absättigen mit PBS-Puffer (2 mM Phosphat, 150 mM NaCl, pH 7,4), der 0,1 % ®Tween 20, pH 7,4 enthielt, wurde die Membran 2 h mit an Peroxidase gekoppeltem Lektin von Griffonia Simplicifolia inkubiert. Nach dem Entfernen ungebundenen Materials mit Hilfe von TBS-Puffer (10 mM Tris, 150 mM NaCl, pH 7,4) wurde die Nitrozellulose 5 - 10 min in 50 mM Tris, 150 mM NaCl, 0,02 % H$_2$O$_2$ und 0,5 mg/ml 4-Chlor-1-naphthol inkubiert. Positive Reaktionen wurden durch Blaufärbung der Proteinbande innerhalb von 5 min sichtbar. Die Reaktion wurde dann durch Transferieren der Nitrozellulose-Membran in Wasser gestoppt.

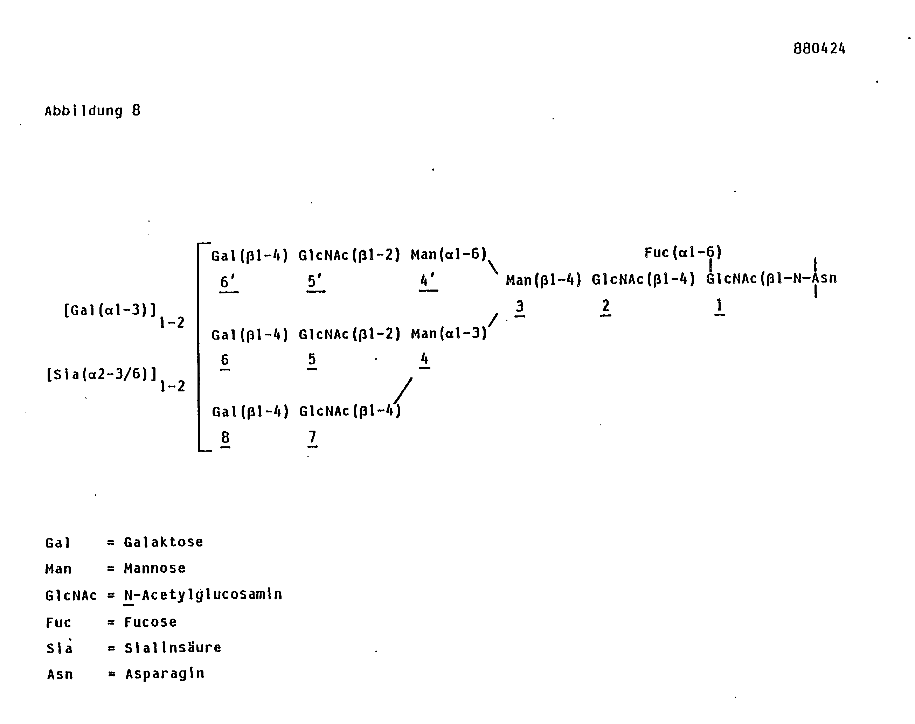

Die Oligosaccharid-Reste enthalten α-gebundene Galaktose-Reste, die gemäß Abb. 8 mit β-gebundenen subterminalen Galaktose-Resten verknüpft sind. Dabei sind die α-Galaktose-Reste bevorzugt an der Galaktose 6′ (Numerierung der Zuckerreste siehe Abb. 8) lokalisiert. Die anderen subterminalen Galaktose-Reste sind entweder durch Sialinsäure oder weitere α-gebundene Galaktose substituiert.

Ansprüche

1. Polypeptide der Formel
tPA$_{1-54}$-X-tPA$_{172-527}$ ,
worin
tPA$_{1-54}$ und tPA$_{172-527}$ die Aminosäuren 1-54 und 172-527 des humanen, gereiften Gewebe-Plasminogenaktivators und
X eine der Aminosäuresequenzen
- Ser-tPA$_{111-171}$
- tPA$_{55-105}$
- tPA$_{55-130}$-Arg-tPA$_{140-171}$ oder
- tPA$_{55-125}$-tPA$_{139-171}$
bedeuten, wobei tPA$_{111-171}$, tPA$_{55-105}$, tPA$_{55-130}$, tPA$_{140-171}$, tPA$_{55-125}$ und tPA$_{139-171}$ die Aminosäuren 111-171, 55-105, 55-130, 140-171, 55-125 und 139-171 des humanen, gereiften Gewebeplasminogenaktivators sind, sowie deren Allelvarianten oder Derivaten mit am N- und/oder C-Terminus um ein bis sechs Aminosäuren verkürzten oder verlängerten Sequenzen.

2. DNA-Sequenzen, die für die Polypeptide gemäß Anspruch 1 kodieren.

3. Vektoren, die Gensequenzen enthalten, die für die Polypeptide gemäß Anspruch 1 kodieren.

4. Arzneimittel enthaltend mindestens ein Polypeptid gemäß Anspruch 1, gegebenenfalls in einem pharmazeutisch verträglichen Träger oder Bindemittel.

5. Arzneimittel nach Anspruch 4, enthaltend die Kombination aus mindestens einem Polypeptid gemäß Anspruch 1 und einem anderen Fibrinolytikum.

Patentanspruch für folgende Vertragsstaat: ES

Gentechnologisches Verfahren zur Herstellung von Polypeptiden der Formel
tPA$_{1-54}$-X-tPA$_{172-527}$ ,
worin
tPA$_{1-54}$ und tPA$_{172-527}$ die Aminosäuren 1-54 und 172-527 des humanen, gereiften Gewebe-Plasminogenaktivators und
X eine der Aminosäuresequenzen
- Ser-tPA$_{111-171}$

- tPA$_{55-105}$
- tPA$_{55-130}$-Arg-tPA$_{140-171}$ oder
- tPA$_{55-125}$-tPA$_{139-171}$

bedeuten, wobei tPA$_{111-171}$, tPA$_{55-105}$, tPA$_{55-130}$, tPA$_{140-171}$, tPA$_{55-125}$ und tPA$_{139-171}$ die Aminosäuren 111-171, 55-105, 55-130, 140-171, 55-125 und 139-171 des humanen, gereiften Gewebeplasminogenaktivators sind, sowie deren Allelvarianten oder Derivaten mit am N- und/oder C-Terminus um ein bis sechs Aminosäuren verkürzten oder verlängerten Sequenzen, dadurch gekennzeichnet, daß man in einem Wirtsorganismus ein Gen zur Expression bringt, das für die Peptidsequenzen nach Anspruch 1 kodiert.

Abb. 1a

```
    ATGGATGCAATGAAGAGAGGGCTCTGCTGTGTGCTGCTGCTGTGTGGAGCAGTCTTCGTT
  1 ---------+---------+---------+---------+---------+---------+ 60
    TACCTACGTTACTTCTCTCCCGAGACGACACACGACGACGACACACCTCGTCAGAAGCAA
```

a:   MetAspAlaMetLysArgGlyLeuCysCysValLeuLeuLeuCysGlyAlaValPheVal  -

                              B
                              g
                              l
                              I
                              I
```
    TCGCCCAGCCAGGAAATCCATGCCCGATTCAGAAGAGGAGCCAGATCTTACCAAGTGATC
 61 ---------+---------+---------+---------+---------+---------+ 120
    AGCGGGTCGGTCCTTTAGGTACGGGCTAAGTCTTCTCCTCGGTCTAGAATGGTTCACTAG
```

a:   SerProSerGlnGluIleHisAlaArgPheArgArgGlyAlaArgSerTyrGlnValIle  -

```
    TGCAGAGATGAAAAAACGCAGATGATATACCAGCAACATCAGTCATGGCTGCGCCCTGTG
121 ---------+---------+---------+---------+---------+---------+ 180
    ACGTCTCTACTTTTTTGCGTCTACTATATGGTCGTTGTAGTCAGTACCGACGCGGGACAC
```

a:   CysArgAspGluLysThrGlnMetIleTyrGlnGlnHisGlnSerTrpLeuArgProVal  -

```
    CTCAGAAGCAACCGGGTGGAATATTGCTGGTGCAACAGTGGCAGGGCACAGTGCCACTCA
191 ---------+---------+---------+---------+---------+---------+ 240
    GAGTCTTCGTTGGCCCACCTTATAACGACCACGTTGTCACCGTCCCGTGTCACGGTGAGT
```

a:   LeuArgSerAsnArgValGluTyrCysTrpCysAsnSerGlyArgAlaGlnCysHisSer  -

```
    GTGCCTGTCAAAAGTTGCAGCGAGCCAAGGTGTTTCAACGGGGGCACCTGCCAGCAGGCC
241 ---------+---------+---------+---------+---------+---------+ 300
    CACGGACAGTTTTCAACGTCGCTCGGTTCCACAAAGTTGCCCCCGTGGACGGTCGTCCGG
```

a:   ValProValLysSerCysSerGluProArgCysPheAsnGlyGlyThrCysGlnGlnAla  -

```
    CTGTACTTCTCAGATTTCGTGTGCCAGTGCCCCGAAGGATTTGCTGGGAAGTGCTGTGAA
301 ---------+---------+---------+---------+---------+---------+ 360
    GACATGAAGAGTCTAAAGCACACGGTCACGGGGCTTCCTAAACGACCCTTCACGACACTT
```

a:   LeuTyrPheSerAspPheValCysGlnCysProGluGlyPheAlaGlyLysCysCysGlu  -

```
    ATAGATACCAGGGCCACGTGCTACGAGGACCAGGGCATCAGCTACAGGGGCACGTGGAGC
361 ---------+---------+---------+---------+---------+---------+ 420
    TATCTATGGTCCCGGTGCACGATGCTCCTGGTCCCGTAGTCGATGTCCCCGTGCACCTCG
```

a:   IleAspThrArgAlaThrCysTyrGluAspGlnGlyIleSerTyrArgGlyThrTrpSer  -

                              H
                              a
                              e
                              I
                              I
```
    ACAGCGGAGAGTGGCGCCGAGTGCACCAACTGGAACAGCAGCGCGTTGGCCCAGAAGCCC
421 ---------+---------+---------+---------+---------+---------+ 480
    TGTCGCCTCTCACCGCGGCTCACGTGGTTGACCTTGTCGTCGCGCAACCGGGTCTTCGGG
```

a:   ThrAlaGluSerGlyAlaGluCysThrAsnTrpAsnSerSerAlaLeuAlaGlnLysPro  -

Abb. 1b

```
     TACAGCGGGCGGAGGCCAGACGCCATCAGGCTGGGCCTGGGGAACCACAACTACTGCAGA
481  ---------+---------+---------+---------+---------+---------+ 540
     ATGTCGCCCGCCTCCGGTCTGCGGTAGTCCGACCCGGACCCCTTGGTGTTGATGACGTCT

a:   TyrSerGlyArgArgProAspAlaIleArgLeuGlyLeuGlyAsnHisAsnTyrCysArg  -


     AACCCAGATCGAGACTCAAAGCCCTGGTGCTACGTCTTTAAGGCGGGGAAGTACAGCTCA
541  ---------+---------+---------+---------+---------+---------+ 600
     TTGGGTCTAGCTCTGAGTTTCGGGACCACGATGCAGAAATTCCGCCCCTTCATGTCGAGT

a:   AsnProAspArgAspSerLysProTrpCysTyrValPheLysAlaGlyLysTyrSerSer  -


     GAGTTCTGCAGCACCCCTGCCTGCTCTGAGGGAAACAGTGACTGCTACTTTGGGAATGGG
601  ---------+---------+---------+---------+---------+---------+ 660
     CTCAAGACGTCGTGGGGACGGACGAGACTCCCTTTGTCACTGACGATGAAACCCTTACCC

a:   GluPheCysSerThrProAlaCysSerGluGlyAsnSerAspCysTyrPheGlyAsnGly  -


     TCAGCCTACCGTGGCACGCACAGCCTCACCGAGTCGGGTGCCTCCTGCCTCCCGTGGAAT
661  ---------+---------+---------+---------+---------+---------+ 720
     AGTCGGATGGCACCGTGCGTGTCGGAGTGGCTCAGCCCACGGAGGACGGAGGGCACCTTA

a:   SerAlaTyrArgGlyThrHisSerLeuThrGluSerGlyAlaSerCysLeuProTrpAsn  -


     TCCATGATCCTGATAGGCAAGGTTTACACAGCACAGAACCCCAGTGCCCAGGCACTGGGC
721  ---------+---------+---------+---------+---------+---------+ 780
     AGGTACTAGGACTATCCGTTCCAAATGTGTCGTGTCTTGGGGTCACGGGTCCGTGACCCG

a:   SerMetIleLeuIleGlyLysValTyrThrAlaGlnAsnProSerAlaGlnAlaLeuGly  -


     CTGGGCAAACATAATTACTGCCGGAATCCTGATGGGGATGCCAAGCCCTGGTGCCACGTG
781  ---------+---------+---------+---------+---------+---------+ 840
     GACCCGTTTGTATTAATGACGGCCTTAGGACTACCCCTACGGTTCGGGACCACGGTGCAC

a:   LeuGlyLysHisAsnTyrCysArgAsnProAspGlyAspAlaLysProTrpCysHisVal  -


     CTGAAGAACCGCAGGCTGACGTGGGAGTACTGTGATGTGCCCTCCTGCTCCACCTGCGGC
841  ---------+---------+---------+---------+---------+---------+ 900
     GACTTCTTGGCGTCCGACTGCACCCTCATGACACTACACGGGAGGACGAGGTGGACGCCG

a:   LeuLysAsnArgArgLeuThrTrpGluTyrCysAspValProSerCysSerThrCysGly  -


     CTGAGACAGTACAGCCAGCCTCAGTTTCGCATCAAAGGAGGGCTCTTCGCCGACATCGCC
901  ---------+---------+---------+---------+---------+---------+ 960
     GACTCTGTCATGTCGGTCGGAGTCAAAGCGTAGTTTCCTCCCGAGAAGCGGCTGTAGCGG

a:   LeuArgGlnTyrSerGlnProGlnPheArgIleLysGlyGlyLeuPheAlaAspIleAla  -


     TCCCACCCCTGGCAGGCTGCCATCTTTGCCAAGCACAGGAGGTCGCCCGGAGAGCGGTTC
961  ---------+---------+---------+---------+---------+---------+ 1020
     AGGGTGGGGACCGTCCGACGGTAGAAACGGTTCGTGTCCTCCAGCGGGCCTCTCGCCAAG

a:   SerHisProTrpGlnAlaAlaIlePheAlaLysHisArgArgSerProGlyGluArgPhe  -
```

O.Z. 0050/40178

Abb. 1c

```
      CTGTGCGGGGGCATACTCATCAGCTCCTGCTGGATTCTCTCTGCCGCCCACTGCTTCCAG
1021  ---------+---------+---------+---------+---------+---------+ 1080
      GACACGCCCCCGTATGAGTAGTCGAGGACGACCTAAGAGAGACGGCGGGTGACGAAGGTC

  a:  LeuCysGlyGlyIleLeuIleSerSerCysTrpIleLeuSerAlaAlaHisCysPheGln -

      GAGAGGTTTCCGCCCCACCACCTGACGGTGATCTTGGGCAGAACATACCGGGTGGTCCCT
1081  ---------+---------+---------+---------+---------+---------+ 1140
      CTCTCCAAAGGCGGGGTGGTGGACTGCCACTAGAACCCGTCTTGTATGGCCCACCAGGGA

  a:  GluArgPheProProHisHisLeuThrValIleLeuGlyArgThrTyrArgValValPro -

      GGCGAGGAGGAGCAGAAATTTGAAGTCGAAAAATACATTGTCCATAAGGAATTCGATGAT
1141  ---------+---------+---------+---------+---------+---------+ 1200
      CCGCTCCTCCTCGTCTTTAAACTTCAGCTTTTTATGTAACAGGTATTCCTTAAGCTACTA

  a:  GlyGluGluGluGlnLysPheGluValGluLysTyrIleValHisLysGluPheAspAsp -

      GACACTTACGACAATGACATTGCGCTGCTGCAGCTGAAATCGGATTCGTCCCGCTGTGCC
1201  ---------+---------+---------+---------+---------+---------+ 1260
      CTGTGAATGCTGTTACTGTAACGCGACGACGTCGACTTTAGCCTAAGCAGGGCGACACGG

  a:  AspThrTyrAspAsnAspIleAlaLeuLeuGlnLeuLysSerAspSerSerArgCysAla -

      CAGGAGAGCAGCGTGGTCCGCACTGTGTGCCTTCCCCCGGCGGACCTGCAGCTGCCGGAC
1261  ---------+---------+---------+---------+---------+---------+ 1320
      GTCCTCTCGTCGCACCAGGCGTGACACACGGAAGGGGGCCGCCTGGACGTCGACGGCCTG

  a:  GlnGluSerSerValValArgThrValCysLeuProProAlaAspLeuGlnLeuProAsp -

      TGGACGGAGTGTGAGCTCTCCGGCTACGGCAAGCATGAGGCCTTGTCTCCTTTCTATTCG
1321  ---------+---------+---------+---------+---------+---------+ 1380
      ACCTGCCTCACACTCGAGAGGCCGATGCCGTTCGTACTCCGGAACAGAGGAAAGATAAGC

  a:  TrpThrGluCysGluLeuSerGlyTyrGlyLysHisGluAlaLeuSerProPheTyrSer -

      GAGCGGCTGAAGGAGGCTCATGTCAGACTGTACCCATCCAGCCGCTGCACATCACAACAT
1381  ---------+---------+---------+---------+---------+---------+ 1440
      CTCGCCGACTTCCTCCGAGTACAGTCTGACATGGGTAGGTCGGCGACGTGTAGTGTTGTA

  a:  GluArgLeuLysGluAlaHisValArgLeuTyrProSerSerArgCysThrSerGlnHis -

      TTACTTAACAGAACAGTCACCGACAACATGCTGTGTGCTGGAGACACTCGGAGCGGCGGG
1441  ---------+---------+---------+---------+---------+---------+ 1500
      AATGAATTGTCTTGTCAGTGGCTGTTGTACGACACACGACCTCTGTGAGCCTCGCCGCCC

  a:  LeuLeuAsnArgThrValThrAspAsnMetLeuCysAlaGlyAspThrArgSerGlyGly -

      CCCCAGGCAAACTTGCACGACGCCTGCCAGGGCGATTCGGGAGGCCCCCTGGTGTGTCTG
1501  ---------+---------+---------+---------+---------+---------+ 1560
      GGGGTCCGTTTGAACGTGCTGCGGACGGTCCCGCTAAGCCCTCCGGGGGACCACACAGAC

  a:  ProGlnAlaAsnLeuHisAspAlaCysGlnGlyAspSerGlyGlyProLeuValCysLeu -
```

Abb. 1d

```
      AACGATGGCCGCATGACTTTGGTGGGCATCATCAGCTGGGGCCTGGGCTGTGGACAGAAG
 1561 ---------+---------+---------+---------+---------+---------+ 1620
      TTGCTACCGGCGTACTGAAACCACCCGTAGTAGTCGACCCCGGACCCGACACCTGTCTTC
```

a:    AsnAspGlyArgMetThrLeuValGlyIleIleSerTrpGlyLeuGlyCysGlyGlnLys -

```
      GATGTCCCGGGTGTGTACACCAAGGTTACCAACTACCTAGACTGGATTCGTGACAACATG
 1621 ---------+---------+---------+---------+---------+---------+ 1680
      CTACAGGGCCCACACATGTGGTTCCAATGGTTGATGGATCTGACCTAAGCACTGTTGTAC
```

a:    AspValProGlyValTyrThrLysValThrAsnTyrLeuAspTrpIleArgAspAsnMet -

```
      CGACCGTGA
 1681 --------- 1689
      GCTGGCACT
```

a:    ArgProEnd -

# Abb.2

1. Schnittstelle für BstX1-Mutanten
2. Schnittstellen für EGF-Mutante

561/88

# Abb. 3

Strategie für BstX1-Mutanten

561/88

# Abb. 4

Strategie für EGF-Mutante

561/88

20

# Abb.5

# Abb. 6

# Abb. 7

Abbildung 8

$$\begin{array}{l}
[\text{Gal}(\alpha1-3)]_{1-2} \\
[\text{Sia}(\alpha2-3/6)]_{1-2}
\end{array}
\begin{bmatrix}
\begin{array}{l}
\underset{\underline{6'}}{\text{Gal}(\beta1-4)} \; \underset{\underline{5'}}{\text{GlcNAc}(\beta1-2)} \; \underset{\underline{4'}}{\text{Man}(\alpha1-6)} \searrow \\
\\
\underset{\underline{6}}{\text{Gal}(\beta1-4)} \; \underset{\underline{5}}{\text{GlcNAc}(\beta1-2)} \; \underset{\underline{4}}{\text{Man}(\alpha1-3)} \nearrow \\
\\
\underset{\underline{8}}{\text{Gal}(\beta1-4)} \; \underset{\underline{7}}{\text{GlcNAc}(\beta1-4)} \nearrow
\end{array}
\end{bmatrix}
\begin{array}{l}
\text{Fuc}(\alpha1-6) \\
\hspace{2em}\Big| \\
\underset{\underline{3}}{\text{Man}(\beta1-4)} \; \underset{\underline{2}}{\text{GlcNAc}(\beta1-4)} \; \underset{\underline{1}}{\text{GlcNAc}}(\beta1-N-\overset{\big|}{\underset{\big|}{\text{Asn}}}
\end{array}$$

Gal     = Galaktose

Man     = Mannose

GlcNAc = N-Acetylglucosamin

Fuc     = Fucose

Sia     = Sialinsäure

Asn     = Asparagin

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 242 836  (BOEHRINGER MANNHEIM) <br> * Ansprüche 1,4,5,29 * <br> --- | 1-4 | C 12 N    9/64 <br> C 12 N   15/58 <br> A 61 K   37/547 |
| Y | EP-A-0 241 208  (BEECHAM GROUP PLC) <br> * Ansprüche 1-7,12-15,21,22,24-29 * <br> --- | 1-4 | |
| Y | THE EMBO JOURNAL, Band 7, Nr. 9, Seite 2731-2740, 25. Aug 1988, M.J. GETHING et al.: "Variants of human tissue-type plasminogen activator that lack specific structural domains of the heavy chain" <br> * Ganzer Artikel * <br> ----- | 1-4 | |
| | | | **RECHERCHIERTE SACHGEBIETE (Int. Cl.5)** <br><br> C 12 N |

Der vorliegende Recherchenbericht wurde für alle Patentansprüche erstellt

| Recherchenort | Abschlußdatum der Recherche | Prüfer |
|---|---|---|
| DEN HAAG | 30-01-1990 | HUBER-MACK A. |